(19) Europäisches Patentamt / European Patent Office / Office européen des brevets

(11) **EP 1 394 071 B1**

(12) **EUROPEAN PATENT SPECIFICATION**

(45) Date of publication and mention
of the grant of the patent:
**26.12.2012 Bulletin 2012/52**

(21) Application number: **02733353.3**

(22) Date of filing: **06.06.2002**

(51) Int Cl.:
*B65D 81/32* (2006.01)     *B65D 30/22* (2006.01)
*A61J 1/05* (2006.01)      *B32B 27/32* (2006.01)
*C08J 5/18* (2006.01)      *C08L 23/10* (2006.01)
*A61J 1/00* (2006.01)

(86) International application number:
**PCT/JP2002/005610**

(87) International publication number:
**WO 2002/100738 (19.12.2002 Gazette 2002/51)**

(54) **DUPLEX CONTAINER**

**DOPPELBEHÄLTER**

**CONTENANT A DEUX COMPARTIMENTS**

(84) Designated Contracting States:
**DE**

(30) Priority:  **07.06.2001   JP 2001172705**

(43) Date of publication of application:
**03.03.2004   Bulletin 2004/10**

(73) Proprietors:
• **Mitsubishi Chemical Corporation**
**Chiyoda-ku**
**Tokyo 100-8251 (JP)**
• **Q.P. Corporation**
**Shibuya-ku,**
**Tokyo 150-0002 (JP)**

(72) Inventors:
• **SAITO, Hisatoshi**
**Kawasaki-shi, Kanagawa 216-0003 (JP)**

• **NISHIHARA, Yoshio**
**Kyoto-shi,**
**Kyoto 612-8006 (JP)**
• **TAKAYANAGI, Kenjiro**
**Yokkaichi-shi,**
**Mie 510-8530 (JP)**

(74) Representative: **HOFFMANN EITLE**
**Patent- und Rechtsanwälte**
**Arabellastrasse 4**
**81925 München (DE)**

(56) References cited:
EP-A- 0 483 523      EP-A- 1 106 644
EP-A- 1 106 647      WO-A-01/35898
JP-A- 7 285 201      JP-A- 8 229 099
JP-A- 9 131 838      JP-A- 2000 005 276
JP-A- 2000 007 050   JP-A- 2001 226 499
JP-B2- 7 000 371     US-A- 4 211 852
US-A- 5 176 634

**EP 1 394 071 B1**

**Description**

TECHNICAL FIELD

The present invention relates to a multi-chamber container, and more particularly to a multi-chamber container comprising a resin film or sheet formed into a bag shape by heat-sealing and having a partition portion formed therein.

BACKGROUND ART

Conventionally, there have been proposed many packaging containers for filling and packaging various products therein. As one of these packaging containers, there have been proposed multi-chamber containers comprising a resin film or sheet formed into a bag shape by heat-sealing and having a partition portion formed therein in which resin film or sheets constituting the partition portion formed by heat-sealing are peelable from each other (for example, Utility Model Application Laid-Open (JIKKAI) Nos. 56-53062, 57-105272 and 3-66845). Such multi-chamber containers have been used for filling and packaging various products comprising two or more components which are mixed together immediately before use thereof, for example, foods and beverages such as retort foods, drugs, cosmetics, chemical products such as adhesives, sundries such as disposable body warmers.

In particular, in the field of drugs, a plurality of drug components is usually administrated together in a mixed state into living organisms. In this case, the above-mentioned multi-chamber containers have been extensively used in order to conduct the mixing procedure under a sterile condition, i.e., in a so-called closed system, or facilitate the mixing procedure, (for example, Japanese Patent Publication (KOKOKU) No. 6-26563, and Japanese Patent Application Laid-Open (KOKAI) Nos. 63-309263, 1-240469, 2-4671, 2-57584, 2-241457, 2-255418, 4-242647, 5-31153, 5-68702, 8-131515, 8-229100 and 2000-70331).

In the case of the above-mentioned multi-chamber containers, peripheral portions thereof which are communicated with an outside (atmospheric air) are required to have a sufficient heat-seal strength, and further it is important that the partition portion provided within the containers is substantially free from peeling-off between resin films or sheets constituting the same upon production and transportation thereof, and exhibit a heat-seal strength to such an extent that the resin films or sheets are readily peeled off from each other by hands or suitable equipments upon use (mixing).

Therefore, it is also important to select an adequate material for the heat-sealed portions of the multi-chamber container. At present, as the materials of the heat-sealed portions, there have been used polyethylene/polypropylene compositions, polyethylene/crosslinked polyethylene compositions.

However, when the above conventional materials are used, it may be difficult to provide a sufficient difference in heat-seal strength between the partition portion and peripheral portions of the container owing to the narrow range of suitable heat-sealing conditions. For this reason, when the heat-seal strength of the container is appropriately controlled in view of the peelability between the resin films or sheets of the partition portion, the heat-seal strength of the peripheral portions tends to be lowered. On the contrary, when the heat-seal strength of the container is increased in view of the heat-seal strength of the peripheral portions, the peelability between the resin films or sheets of the partition portion tends to be deteriorated.

For example, in the case of the multi-chamber containers proposed in the above Japanese Patent Application Laid-Open (KOKAI) Nos. 8-131515, 8-229100 and 2000-70331, the difference in heat-seal strength between the partition portion and peripheral portions of the container is as small as merely about 2 kgf/15 mm.

Also, in the case of the conventional materials, since the heat-sealing temperature range capable of forming the peelable partition portion is generally as narrow as 10°C or lower, there arises such a problem that a slight change of the heat-sealing temperature tends to cause extreme change in heat-seal strength. Therefore, upon formation of the partition portion, it may become very difficult to control the temperature of a heat-sealing mold used therefor, resulting in high occurrence rate of defective products. This is because the mass production of the multi-chamber containers is inhibited.

Further, in the case of multi-chamber containers used for medical purposes, it is necessary not only to solve the above problems concerning the heat-seal strength between the partition portion and peripheral portions of the container, but also to satisfy the requirements including safety, flexibility, transparency, heat resistance (heat and pressure resistance upon sterilization). For example, the polyethylene/polypropylene compositions have problems such as insufficient transparency and heat resistance.

JP-A-2000-005276 concerns a container for a medicament having a separable seal section at a heat-bonding part of a resin container. The interior of the container is separated into more than two chambers by the seal section. The container wall of the container is formed of a base layer and an innermost layer. The innermost layer is formed of a resin composition comprising a syndiotactic polypropylene, a propylene-ethylene random copolymer or a specific polypropylene.

US 5,176,634 concerns a flexible container for the storage and mixing together of diluents and medicaments.

2

The container incorporates multiple compartments, separated by frangible seals, in which the diluents and medicaments are stored. The seals are ruptured by manipulation of the container to thereby mix the contents together for delivery through a standard intravenous arrangement to a patient.

WO 01/35898 is directed to a container comprising a body made from a film. The body includes at least one side seal. At least two chambers are separated in the container by a peelable seal. The film includes a sealant layer having a bimodal thermal behavior such that the side seal is a permanent seal and the peelable seal can be separated.

EP 0 483 523 A1 concerns compositions of crystalline propylene polymers comprising 30-65 % by weight of a copolymer of 98 to 80 % of propylene with a $C_4$-$C_8$ $\alpha$-olefin and 35-70 % by weight of a copolymer of propylene with ethylene and optionally 2 to 10 % of a $C_4$-$C_8$ $\alpha$-olefin.

US 4,211,852 concerns a thermoplastic olefin resin composition composed of a blend of 5 to 95 % by weight of a random copolymer consisting essentially of 55 to 85 mole % of propylene and 45 to 15 mole % of 1-butene and having a heat of fusion of 10 to 80 J/g (DSC) and 5 to 95 % by weight of an isotactic propylene resin having a melting point of 135 to 165°C and a melt index of 0.1 to 20 (at 230°C).

In view of the above problems, an object of the present invention is to provide a multi-chamber container comprising a resin film or sheet formed into a bag shape by heat-sealing and having a partition portion provided therein which is capable of showing a large difference in heat-seal strength between the partition portion and peripheral portions of the container as compared to conventional ones, readily controlling the temperature of a heat-sealing mold upon formation of the partition portion, and further exhibiting excellent safety, flexibility, transparency and heat resistance so as to be usable in medical applications.

BRIEF DESCRIPTION OF THE DRAWING

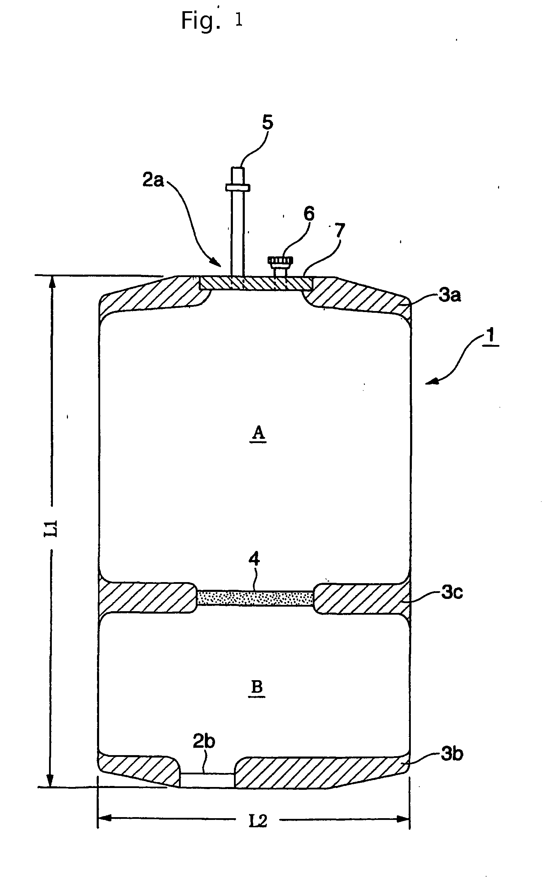

Fig. 1 is an explanatory plan view showing an example of a multi-chamber container according to the present invention in which reference numeral 1 represents a body of a multi-chamber container for medical use; 2a, 2b: filling ports for liquid drug; 3a, 3b, 3c: strong sealed portions; 4: weak sealed portion; 5: filling and discharging port for liquid drug; 6: mixing and pouring port; 7: ship-shaped port member; and A, B: accommodation chambers.

DISCLOSURE OF THE INVENTION

As a result of the present inventors' earnest studies for solving the above problems, it has been found that when a specific propylene-based resin composition is used as a material for a heat-sealed portion of the multi-chamber container, the above object can be readily accomplished. The present invention has been attained on the basis of this finding.

That is, according to a aspect of the present invention, there is provided a multi-chamber container comprising a resin film or sheet formed into a bag shape by heat-sealing and having a partition portion formed therein, said multi-chamber container including a heat-sealed portion comprising a propylene-based resin composition according to claims 1 to 4.

According to a preferable aspect of the present invention, the propylene-based resin composition satisfies such a requirement that a difference between a heat-sealing temperature (T1) (°C) providing a heat-seal strength of 0.2 kgf/15 mm and a heat-sealing temperature (T2) (°C) providing a heat-seal strength of 2.0 kgf/15 mm (T2 - T1) is not less than 20°C, and exhibiting a heat-seal strength of not less than 3 kgf/15 mm.

The present invention will be described in detail below. The multi-chamber container according to the present invention may have a configuration, for example, as shown in Fig. 1, though not particularly limited thereto. The multi-chamber container shown in Fig. 1 is a medical multi-chamber container produced by forming a cylindrical film body obtained by inflation method into a bag shape by known methods, and has the following structure and functions.

That is, a container body (1) is provided at opposite end portions thereof with strong sealed portions (3a) and (3b) so as to leave filling ports (2a) and (2b) for liquid drug, and at a position slightly lower than the mid of the container body (1) with a partition portion separating an interior of the container into an upper accommodation chamber (A) and a lower accommodation chamber (B). The partition portion includes a strong sealed portion (3c) extending inwardly from a side wall of the container (i.e., from right and left sides as viewed in Fig. 1) and a weak sealed portion (4) formed inside the strong sealed portion (3c) (i.e., between the right and left side portions (3c) as viewed in Fig. 1). To the filling port (2a) is welded a boat-shaped port member (7) to which a filling and discharging pipe (5) and a mixing and pouring port (6) are connected. When the accommodation chambers (A) and (B) are pressed by hands or equipments upon use, resin films constituting the weak sealed portion (4) of the partition portion are peeled off from each other, so that the accommodation chambers (A) and (B) are communicated with each other to enable drug components isolated from each other by the partition portion to be readily mixed together.

<u>\<Multi-chamber container according to the present invention</u>

First, the multi-chamber container of the inventive concept of the present invention is explained. The inventive concept of the present invention is characterized in that the heat-sealed portion of the multi-chamber container is comprising a propylene-based resin composition according to claim1 containing the following component (A) obtained by a first-stage polymerization and the following component (B) obtained by a second-stage polymerization such that the components (A) is contained in an amount of 10 to 60% by weight and the components (B) is contained in an amount of 40 to 90% by weight, based on a total weight of the components (A) and (B):

Component (A): a polymer component containing propylene having an isotactic index of not less than 90% as a main component; and
Component (B): a copolymer component comprising a copolymer produced from propylene and an $\alpha$-olefin other than propylene having not more than 8 carbon atoms with the proviso that propylene and ethylene are contained therein as essential components, and containing a room-temperature xylene-insoluble component in an amount of from more than 20% by weight to 70% by weight based on a total weight of whole polymers as a sum of the components (A) and (B), and a room-temperature xylene-soluble component in an amount of from 10% by weight to 60% by weight based on a total weight of whole polymers as a sum of the components (A) and (B), said room-temperature xylene-soluble component having a content of the $\alpha$-olefin other than propylene of less than 20% by weight.

The above component (A) is usually composed of a room-temperature xylene-insoluble component (crystalline component) and a room temperature xylene-soluble component (amorphous component). The content of the former crystalline component substantially corresponds to the isotactic index. The "polymer component containing propylene as a main component" means a polymer containing constituting units derived from propylene in an amount of usually not less than 70% by weight. The content of the constituting units derived from propylene is preferably not less than 90% by weight, more preferably not less than 95% by weight, especially preferably 100% by weight (propylene homopolymer). When the isotactic index of the component (A) is less than 90%, the propylene-based resin composition tends to be deteriorated in heat resistance.

Examples of the $\alpha$-olefin other than propylene usable in the above component (B) may include 1-butene, 3-methyl-1-butene, 1-pentene, 4-methyl-1-pentene, 1-hexene, 1-octene or the like. The especially preferred component (B) is a propylene-ethylene copolymer.

The content of the "room-temperature xylene-insoluble component" in the component (B) is in the range of from more than 20 to 70% by weight, preferably from 25 to 65% by weight based on the total weight of whole polymers as a sum of the components (A) and (B). When the content of the "room-temperature xylene-insoluble component" in the component (B) is not more than 20% by weight, the propylene-based resin composition tends to be deteriorated in transparency. When the content of the "room-temperature xylene-insoluble component" in the component (B) is more than 70% by weight, the propylene-based resin composition tends to be deteriorated in flexibility.

The content of the "room-temperature xylene-soluble component" in the component (B) is in the range of from 10 to 60% by weight, preferably from 15 to 60% by weight based on the total weight of whole polymers as a sum of the components (A) and (B). When the content of the "room-temperature xylene-soluble component" in the component (B) is less than 10% by weight, the propylene-based resin composition tends to be deteriorated in flexibility. When the content of the "room-temperature xylene-soluble component" in the component (B) is more than 60% by weight, the propylene-based resin composition tends to be deteriorated in heat resistance.

Also, the content of the $\alpha$-olefin other than propylene in the above "room-temperature xylene-soluble component" is usually less than 20% by weight, preferably 10 to 18% by weight. When the content of the $\alpha$-olefin other than propylene in the above "room-temperature xylene-soluble component" is not less than 20% by weight, the propylene-based resin composition tends to be deteriorated in transparency.

The content of the component (A) is preferably 20 to 50% by weight based on the total weight of the components (A) and (B), and the content of the component (B) is preferably 50 to 80% by weight based on the total weight of the components (A) and (B). When the content of the component (A) is less than 10% by weight and the content of the component (B) is more than 90% by weight, the obtained propylene-based resin composition tends to be deteriorated in heat resistance. When the content of the component (A) is more than 60% by weight and the content of the component (B) is less than 40% by weight, the obtained propylene-based resin composition tends to be insufficient in flexibility and transparency.

The above propylene-based resin composition according to claim 1 also satisfies the following requirements (i) to (iv):

(i) a propylene content in whole polymers as a sum of the components (A) and (B) is within the range of 85 to 95% by weight;

(ii) a content of the α-olefin other than propylene (hereinafter referred to merely as "αt"; unit: % by weight) and a content of a room-temperature xylene-soluble component (hereinafter referred to merely as "CXS") in the whole - polymers as a sum of the components (A) and (B) satisfies the following formula:

$$CXS > 5\alpha t - 25$$

wherein αt is 5 to 15% by weight;

(iii) a melting point peak temperature thereof is not less than 160°C; and

(iv) a tensile stress at a yielding point thereof is not more than 15 MPa.

As to the requirement (i), the propylene content and the content of the α-olefin other than propylene having 2 to 8 carbon atoms are preferably in the range of 85 to 95% by weight and 5 to 15% by weight, respectively. The propylene content and the content of the α-olefin other than propylene are more preferably in the range of 87 to 95% by weight and 5 to 13% by weight, respectively, and still more preferably 88 to 92% by weight and 8 to 12% by weight, respectively. When the propylene content exceeds the above-specified range (i.e., the content of the α-olefin other than propylene is less than the above-specified range), the propylene-based resin composition tends to be poor in flexibility. On the other hand, when the propylene content is less than the above-specified range (i.e., the content of the α-olefin other than propylene exceeds the above-specified range), the propylene-based resin composition tends to be deteriorated in transparency.

If the above requirement (ii) is not satisfied, the propylene-based resin composition tends to be deteriorated in transparency.

The propylene-based resin composition preferably used in the inventive concept of the present invention satisfies the above requirements (i) and (ii), and further exhibits such a high heat resistance that a melting point thereof is substantially identical to that of propylene homopolymer and a peak temperature of the melting point is 160°C or higher (requirement (iii)), as well as a sufficient flexibility (requirement (iv)). Meanwhile, the tensile stress at a yielding point represents the value prescribed according to ISO-R1184.

Upon production of the above propylene-based resin composition, the component (A) is produced by a first-stage polymerization, and then the component (B) is produced by a second-stage polymerization. For example, in at least two-stage polymerization, after producing a propylene homopolymer at the first stage, the copolymer of propylene and the α-olefin other than propylene having 2 to 8 carbon atoms which contains propylene and ethylene as essential components, is produced at the second or subsequent stage in the presence of the polymer obtained at the previous stage. The thus obtained propylene-based resin composition preferably has, as a whole, a propylene content of 85 to 95% by weight and a content of the α-olefin other than propylene of 5 to 15% by weight.

Examples of preferred catalysts used in the above successive polymerization reactions may include those catalysts comprising an organoaluminum compound and a solid component containing a titanium atom, a magnesium atom, a halogen atom and an electron donative compound as essential components, though not particularly limited thereto.

As the organoaluminum compound, there may be used compounds represented by the general formula:

$$R^1_m AlX_{(3-m)}$$

wherein $R^1$ is a hydrocarbon residue having 1 to 12 carbon atoms; X is a halogen atom; and m is a number of 1 to 3.

Examples of the organoaluminum compound may include trialkyl aluminums such as trimethyl aluminum and triethyl aluminum, dialkyl aluminum halides such as dimethyl aluminum chloride and diethyl aluminum chloride, alkyl aluminum sesquihalides such as methyl aluminum sesquichloride and ethyl aluminum sesquichloride, alkyl aluminum dihalides such as methyl aluminum dichloride and ethyl aluminum dichloride, alkyl aluminum hydrides such as diethyl aluminum hydride.

As titanium compounds as a supply source of the titanium atom in the above solid component, there may be used compounds represented by the general formula:

$$Ti(OR^2)_{(4-n)}X_n$$

wherein $R^2$ is a hydrocarbon residue having 1 to 10 carbon atoms; X is a halogen atom; and n is a number of 0 to 4.

Among these titanium compounds, preferred are titanium tetrachloride, tetraethoxy titanium, tetrabutoxy titanium .

Examples of magnesium compounds as a supply source of the magnesium atom in the solid component may include dialkylmagnesiums, magnesium dihalides, dialkoxymagnesiums, alkoxymagnesium halides. Among these magnesium compounds, preferred are magnesium dihalides or the like. As the halogen atom, there may be used fluorine, chlorine and iodine. Among these halogens, preferred is chlorine. The halogen atom may be usually supplied from the above titanium compounds or magnesium compounds, and may also be supplied from other halogen supply sources such as aluminum halides, silicon halides and tungsten halides.

Examples of the electron donative compound may include oxygen-containing compounds such as alcohols, phenols, ketones, aldehydes, carboxylic acids, organic or inorganic acids and derivatives thereof; nitrogen-containing compounds such as ammonia, amines, nitriles and isocyanates. Among these compounds, preferred are inorganic acid esters, organic acid esters and organic acid halides, and more preferred are silicic acid esters, phthalic acid esters, cellosolve acetate and phthalic acid halides.

As the above silicic acid esters, there may be used organosilicon compounds represented by the general formula:

$$R^3R^4_{(3-p)}Si(OR^5)_p$$

wherein $R^3$ is a branched aliphatic hydrocarbon residue having 3 to 20 carbon atoms, preferably 4 to 10 carbon atoms, or a cyclic aliphatic hydrocarbon residue having 5 to 20 carbon atoms, preferably 6 to 10 carbon atoms; $R^4$ is a branched or linear aliphatic hydrocarbon residue having 1 to 20 carbon atoms, preferably 1 to 10 carbon atoms; $R^5$ is an aliphatic hydrocarbon residue having 1 to 10 carbon atoms, preferably 1 to 4 carbon atoms; and p is a number of 1 to 3. Specific examples of such organosilicon compounds may include t-butyl-methyl-dimethoxysilane, t-butyl-methyl-diethoxysilane, cyclohexyl-methyl-dimethoxysilane, cyclohexyl-methyl-diethoxysilane.

Upon production of the propylene-based resin composition, propylene, or propylene and the $\alpha$-olefin other than propylene having 2 to 8 carbon atoms are supplied at the first stage, and then the $\alpha$-olefin containing propylene as a main component is polymerized in the presence of the above catalyst at a temperature of 50 to 150°C, preferably 50 to 100°C under a propylene partial pressure of 0.5 to 4.5 MPa, preferably 1.0 to 3.5 MPa, thereby producing the component (A). Successively, at the second stage, propylene and ethylene, or propylene, ethylene and the other $\alpha$-olefin having 4 to 8 carbon atoms, are supplied, and then copolymerized in the presence of the above catalyst at a temperature of 50 to 150°C, preferably 50 to 100°C while controlling each of propylene and ethylene partial pressures to 0.3 to 4.5 MPa, preferably 0.5 to 3.5 MPa, thereby conducting copolymerization between propylene and ethylene or between propylene, ethylene and the $\alpha$-olefin to produce the component (B).

The above polymerization processes may be respectively conducted by any of batch, continuous and semi-batch methods. The first-stage polymerization may be preferably conducted in either a gas phase or a liquid phase, and the second or subsequent stage polymerization may also be preferably in either a gas phase or a liquid phase, more preferably in a gas phase. The residence time of the respective stages may be 0.5 to 10 hours, preferably 1 to 5 hours.

In the above polymerization processes, the contents of the components (A) and (B) may be controlled by appropriately selecting the amounts of monomers to be polymerized at the respective stages, and the isotactic index of the component (A) may be controlled by the kind of catalyst used, polymerization conditions (such as temperature and pressure) or compositions of monomers charged. Also, the contents of the "room-temperature xylene-insoluble component" and the "room-temperature xylene-soluble component" in the component (B) may be controlled by compositions of monomers charged at the respective polymerization stages, amounts of polymers produced at the respective states, for example, the molecular weight that can be adjusted by the amount of hydrogen supplied, , as well as the kind of catalyst used. Further, the respective conditions for the above requirements (ii) and (iii) may be controlled by the ratio between amount of polymers obtained in the first stage and that obtained in the second or subsequent stage, for example, the molecular weight that can be adjusted by the amount of hydrogen supplied.

In the case where particles of the propylene-based resin composition produced by the above method suffer from problems such as stickiness, an active hydrogen-containing compound may be preferably added thereto after the first-stage polymerization, or before or in the course of the second-stage polymerization in order to impart a good flowability to the particles. Examples of the active hydrogen-containing compound may include water, alcohols, phenols, aldehydes, carboxylic acids, acid amides, ammonia, amines. The amount of the active hydrogen-containing compound added is usually 100 to 1,000 mol per mol of the titanium atom contained in the solid component of the catalyst, and 2 to 5 mol per mol of the organoaluminum compound contained in the catalyst.

The propylene-based resin composition may be blended with ethylene-based polymers such as ethylene-$\alpha$-olefin copolymers and ethylene-vinyl acetate copolymers, propylene-based polymers such as propylene-$\alpha$-olefin copolymers and syndiotactic polypropylenes, hydrogenated products of block copolymers of styrene with a conjugated diene such as butadiene and isoprene, unless the blending of these polymers adversely affects the transparency and heat resistance of the composition. Further, an $\alpha$-crystal nucleating agent ordinarily used for improving the transparency of propylene-based polymers may be added to the propylene-based resin composition. In addition, the propylene-based resin composition may be blended with a softening agent for rubbers in order to impart a good flexibility thereto.

Also, the propylene-based resin composition may contain various other resins, fillers such as rubbers, glass fibers, calcium carbonate, silica, talc, mica and clay, and various additives such as antioxidants, light stabilizers, antistatic agents, lubricants, dispersants, neutralizers and flame retardants, if required, unless the addition thereof adversely affects the effect of the inventive concept of the present invention.

The propylene-based resin composition may be used in the form of a single film or sheet. The single film or sheet may be formed, for example, by an inflation method, a T-die method or a calender method. Also, the propylene-based resin composition may be used in the form of a laminated film or sheet. The laminated film or sheet may be formed, for example, by an extrusion-lamination method, a heat-lamination method or a dry-lamination method. The above respective films or sheets may be stretched in monoaxial or biaxial directions. The stretching may be performed, for example, by a roll method, a tenter method or a tubular method.

In the case of the laminated film or sheet, the materials of the layers other than the heat-seal layer (innermost layer of the multi-chamber container) may be appropriately selected from other polymers and compositions according to required properties such as gas-barrier properties to oxygen or water vapor, transparency, flexibility, heat resistance, strength.

For example, in the case where the laminated film or sheet has a three-layer structure, one example of the layer structures satisfying the above required properties when applied to medical multi-chamber containers is as follows. That is, as the material constituting the intermediate layer, there may be used compositions of homopolymers or copolymers of crystalline polypropylene with various elastomers, non- or low-crystalline polypropylenes. Among these materials, especially preferred are resin compositions comprising the propylene-based resin composition with a styrene-based elastomer. In this case, the content of the propylene-based resin composition in the resin composition is 50 to 99% by weight, and the content of the styrene-based elastomer therein is 1 to 50% by weight. Also, as the material constituting the outermost layer (outer wall surface of the container), there may be used a propylene random copolymer resin, or a resin composition composed of the propylene-based resin composition and the propylene random copolymer resin. In this case, the content of the propylene-based resin composition in the resin composition is 5 to 80% by weight, and the content of the propylene random copolymer resin is 20 to 95% by weight.

The above styrene-based elastomer represents a hydrogenated derivative of vinyl aromatic hydrocarbon/conjugated diene block copolymer, and includes one or more hydrogenated derivatives of the block copolymers represented by the general formula:

$$a(b\text{-}a)_n, (a\text{-}b)_n \text{ or } a\text{-}b\text{-}c$$

wherein (a) is a polymer block of a monovinyl-substituted aromatic hydrocarbon; (b) is a random copolymer block of a monovinyl-substituted aromatic hydrocarbon with a conjugated diene, or an elastomeric polymer block of a conjugated diene; (c) is a block of a monovinyl-substituted aromatic hydrocarbon with a conjugated diene which is also in the form of a tapered block in which the monovinyl-substituted aromatic hydrocarbon is gradually increased; n is an integer of 1 to 5.

Examples of the vinyl aromatic hydrocarbon as a monomer constituting the above polymer block (a), (b) or (c) may include styrene, α-methyl styrene, o-, m- or p-methyl styrene, 1,3-dimethyl styrene, vinyl naphthalene, vinyl anthracene . Among these monomers, preferred are styrene and α-methyl styrene. The conjugated diene monomer in the polymer block (b) or (c) preferably includes butadiene and/or isoprene.

In the case where butadiene is used as a single conjugated monomer for forming the polymer block (b) or (c), it is preferable to use such polymerization conditions in which the content of 1,2-microstructure in whole microstructures of polybutadiene is 20 to 50% by weight, more preferably 35 to 45% by weight, for the purpose of maintaining a good elastomeric property even after the block copolymer is hydrogenated to saturate double bonds thereof.

The content of the polymer block (a) in the hydrogenated block copolymer or the total content of the vinyl aromatic compounds in the polymer blocks (a) and (c) is usually 3 to 50% by weight, preferably 5 to 45% by weight, more preferably 5 to 40% by weight. When the content of the polymer block (a) in the hydrogenated block copolymer or the total content of the vinyl aromatic compounds in the polymer blocks (a) and (c) is less than 3% by weight, the obtained thermoplastic elastomer tends to be deteriorated in mechanical strength or - heat resistance. When the content of the polymer block (a) in the hydrogenated block copolymer or the total content of the vinyl aromatic compounds in the polymer blocks (a) and (c) is more than 50% by weight, the styrene-based elastomer tends to be deteriorated in flexibility and rubber elasticity.

In the case where the polymer block (b) is a random copolymer block of a monovinyl-substituted aromatic hydrocarbon and a conjugated diene, the content of vinyl bonds in the conjugated diene moiety is usually more than 60%, preferably not less than 70%, more preferably not less than 80%. When the vinyl bond content is not more than 60%, the effect of improving the flexibility of resins tends to be deteriorated. Meanwhile, the "vinyl bond" used herein represents a monomer unit which is the conjugated diene compound polymerized at its double bond present at the 1,2- or 3,4-bonding position.

The molecular structure of the hydrogenated block copolymer may be in the form of a linear structure, a branched structure, a radial structure or any optional combination thereof. The value (integer) n in the general formula representing these block copolymers is 1 to 5. When the integer n is more than 5, the production process tends to become complicated and uneconomical, and no further remarkable effect is attainable thereby. When the integer n is less than 1, it is not possible to obtain the aimed block copolymer.

The styrene-based elastomer (hydrogenated block copolymer) has a weight-average molecular weight of usually 100,000 to 550,000, preferably 150,000 to 500,000, more preferably 200,000 to 450,000 in terms of polystyrene when measured by gel permeation chromatography. When the weight-average molecular weight is less than 100,000, the styrene-based elastomer tends to be deteriorated in rubber elasticity and mechanical strength. When the weight-average molecular weight is more than 550,000, the styrene-based elastomer tends to show a high viscosity and, therefore, be deteriorated in moldability.

The above styrene-based elastomer (hydrogenated derivative of vinyl aromatic hydrocarbon/conjugated diene block copolymer) can be obtained by polymerization or blending. The method for production of the above block copolymer is not particularly restricted, and the block copolymer may be produced, for example, by the method of conducting the block copolymerization in an inert solvent using a lithium catalyst or the like as described in Japanese Patent Publication (KOKOKU) No. 40-23798, Japanese Patent No. 2764746.

The hydrogenation of the above block copolymer may be performed in an inert solvent using a hydrogenation catalyst, for example, according to the methods described in Japanese Patent Publication (KOKOKU) Nos. 42-8704 and 43-6636, Japanese Patent Application Laid-Open (KOKAI) Nos. 59-133203 and 60-79005. In the hydrogenation, at least 50% by weight, preferably not less than 80% by weight of olefinic double bonds contained in the polymer block (b) are hydrogenated. When the hydrogenation rate is less than 50% by weight, the styrene-based elastomer tends to be deteriorated in weather resistance. Also, not more than 25% by weight of the aromatic unsaturated bonds contained in the polymer block (a) are hydrogenated.

Examples of the above hydrogenated derivative of the vinyl aromatic hydrocarbon/conjugated diene block copolymer may include commercially available products such as "KRATON-G" produced by Shell Chemicals Corp., "SEPTON" and "HYBRAR" produced by Kuraray Co., Ltd., "TUFTEC" produced by Asahi Kasei Co., Ltd., and "DYNARON" produced by JSR Co., Ltd.

On the other hand, the above propylene random copolymer resin represents a random copolymer of propylene with a small amount of $\alpha$-olefin. Here, the $\alpha$-olefin represents an $\alpha$-olefin other than propylene having 2 to 8 carbon atoms. Specific examples of the $\alpha$-olefin may include ethylene, 1-butene, 3-methyl-1-butene, 3-methyl-1-pentene, 4-methyl-1-pentene, 1-octene. Among these $\alpha$-olefins, preferred is ethylene. In addition, the amount of the $\alpha$-olefin copolymerized with propylene is usually 0.1 to 5% by weight, preferably 1.5 to 4% by weight.

The thickness (whole thickness) of the film or sheet forming the multi-chamber container is usually 100 to 600 $\mu$m, preferably 150 to 450 $\mu$m. In the case of the laminated film or sheet, the heat-seal layer comprising the propylene-based composition (innermost layer of the multi-chamber container) has a thickness of usually not less than 10 $\mu$m, preferably not less than 20 $\mu$m, and the upper limit of the thickness of the heat-seal layer is usually 100 $\mu$m.

Further, the surface of the film or sheet forming the multi-chamber container may be roughened in order to prevent occurrence of blocking therebetween. For this purpose, there may also be used anti-blocking agents or slip agents.

The multi-chamber container according to the inventive concept of the present invention may be produced by known bag-making techniques using heat-sealing method. That is, a film or sheet, a parison, of a cylindrical or flat shape may be properly processed and formed into a desired shape or configuration by using an appropriate method such as thermoforming, blowing, stretching, cutting, fusing or the like.

The most important point of the process for production of the multi-chamber container is the heat-sealing step. That is, in the case of the multi-chamber container shown in Fig. 1, the strong sealed portions (3a) to (3c) are required to have a sufficient heat-seal strength. On the other hand, it is important that the weak sealed portion (4) hardly undergoes peeling between resin films or sheets upon production or transportation of the container, but exhibits such a heat-seal strength that the portion is readily peeled off by hands or equipments upon use (upon mixing).

More specifically, the strong sealed portions (3a) to (3c) have a 180° peel strength of usually 3 to 6 kgf/15mm, and the weak sealed portion (4) has a 180° peel strength of usually 0.2 to 2 kgf/15mm. In addition, the difference in heat-seal strength between the strong sealed portions and the weak sealed portion is not less than 3 kgf/15 mm. Such a difference in heat-seal strength may be produced by controlling sealing conditions of the respective sealed portions.

The propylene-based resin composition constituting the heat-sealed layer of the multi-chamber container (innermost layer of the multi-chamber container) according to the inventive concept of the present invention has such an extremely unique property that the heat-sealing temperature and heat-seal strength thereof are substantially proportional to each other over a temperature range as broad as about 50°C. Accordingly, the heat-seal strength of the sealed portions comprising the propylene-based resin composition can be readily varied only by changing the temperature of a heat-sealing mold upon the heat-sealing step. Namely, the weak sealed portion and the strong sealed portions can be readily and surely produced merely by varying the temperature of the heat-sealing mold, and further the difference in heat-seal strength (180°C peel strength) between the weak sealed portion and the strong sealed portions can also be increased to not less than 3 kgf/15 mm.

In addition, since the temperature range capable of forming the weak sealed portion is as broad as not less than 20°C, even though the heat-sealing temperature is fluctuated to some extent, the weak sealed portion can be prevented from undergoing extreme increase or decrease in heat-seal strength thereof. Therefore, even when the temperature of the heat-sealing mold is fluctuated to some extent upon formation of the weak sealed portion, the heat-seal strength of the resultant weak sealed portion can be kept substantially unchanged, thereby ensuring formation of the weak sealed portion. That is, the temperature of the heat-sealing mold can be readily controlled upon mass production of multi-chamber containers, and the occurrence rate of defective products can be reduced.

The ordinary heat-sealing conditions used for production of the multi-chamber container according to the inventive concept of the present invention are as follows. Namely, in the case of the weak sealed portion, the temperature of the heat-sealing mold is about 120 to 140°C, the pressure thereof is about 1 to 5 kgf/cm$^2$, the heat-sealing time is about 1 to 8 seconds, and the heat-sealing width is about 5 to 20 mm. Also, in the case of the strong sealed portions, the temperature of the heat-sealing mold is about 150 to 220°C, the pressure thereof is about 1 to 5 kgf/cm$^2$, the heat-sealing time is about 1 to 10 seconds, and the heat-sealing width is about 5 mm or more.

In the above multi-chamber container according to the inventive concept of the present invention, the resin films or sheets forming the partition portion are peeled off from each other by applying a pressing force thereto from outside upon use. Therefore, at least one of the contents in the container is liquid. The above multi-chamber container according to the inventive concept of the present invention is useful, in particular, for receiving the combination of contents that tend to suffer from deterioration in quality when mixed, such as combination of an amino acid solution and a glucose solution. In the case of the medical multi-chamber container, the number of accommodation chambers is generally 2 to 4.

Further, the present invention is preferably characterized in that the heat-sealed portion of the multi-chamber container is comprising a propylene-based resin composition capable of satisfying such a requirement that a difference between a heat-sealing temperature (T1) (°C) providing a heat-seal strength of 0.2 kgf/15 mm and a heat-sealing temperature (T2) (°C) providing a heat-seal strength of 2.0 kgf/15 mm (T2 - T1) is not less than 20°C, and exhibiting a heat-seal strength of not less than 3 kgf/15 mm.

As described above, the strong sealed portions of the multi-chamber container have a 180° peel strength of usually 3 to 6 kgf/15 mm, and the weak sealed portion thereof has a 180° peel strength of usually 0.2 to 2 kgf/15 mm. Also, the strength of the heat-sealed portion depends upon the heat-sealing temperature under the condition that the heat-sealing time is kept constant.

The meanings of the above thermal properties of the propylene-based resin composition used in the inventive concept are as follows. Namely, the temperature difference (T2-T1) of not less than 20°C means that the heat-sealing temperature range capable of forming the weak sealed portion is broad, i.e.; the rate of increase in heat-seal strength relative to heat-sealing temperature rise is low. Also, the heat-seal strength of not less than 3 kgf/15 mm which is achievable by the propylene-based resin composition means that the strong sealed portions can be formed therefrom.

The strong sealed portions can be relatively easily formed by raising the temperature of the heat-sealing mold. However, in the case where the heat-sealing temperature range capable of forming the weak sealed portion is narrow, it is not easy to form both the strong and weak sealed portions from a single material. The reason therefor is that the slight change in the heat-sealing temperature used for forming the weak sealed portion tends to result in production of not the weak sealed portion but the strong sealed portion.

Further, since the propylene-based resin composition used in the inventive concept has a broad temperature range capable of forming the weak sealed portion, it is possible to increase the difference in heat-seal strength between the weak sealed portion and the strong sealed portions.

PREFERRED EMBODIMENT FOR CARRYING OUT THE INVENTION

The present invention is described in more detail with reference to the following example, but the example is only illustrative and not intended to limit the scope of the present invention thereto. Meanwhile, in the following example and comparative example, "%" represents "% by weight" unless otherwise specified. Further, the evaluation methods used herein are as follows.

<Composition analysis of propylene-based resin composition>

(1) Weight percentage of components (A) and (B) based on whole composition:

The weight percentage of the component (B) based on whole polymers (hereinafter referred to as "B(%)") is calculated from the total weight of the obtained whole polymers and the weights of propylene and ethylene supplied at the second-stage polymerization. On the basis of the thus calculated weight percentage of the component (B), the weight percentage of the component (A) based on whole polymers (hereinafter referred to as "A(%)") is calculated according to the formula "100 - B".

<u>(2) Weight percentage of "room-temperature xylene-soluble component (CXS):</u>

The polymer produced after the first-stage polymerization was sampled, and then 1 g of the sample was added to 300 ml of xylene within an oil bath and dissolved therein at 140°C as a boiling point of xylene under stirring. Then, the resultant solution was continuously stirred for one hour. Successively, the solution was cooled to 100°C under stirring within one hour, and then transferred into an oil bath for rapid cooling where the solution was rapidly cooled to 23±2°C while continuously stirring to precipitate a polymer. Then, the reaction solution was allowed to stand for not less than 20 minutes. The obtained precipitated polymer was naturally filtered by gravity through a filter paper. The resultant filtrate was subjected to evaporation to dryness using an evaporator, dried under reduced pressure at 120°C for 2 hours, and then allowed to stand for cooling to ordinary temperature to measure a weight of the dried product, thereby obtaining an amount of the "room-temperature xylene-soluble component" contained in the component (A). The obtained amount of the "room-temperature xylene-soluble component" contained in the component (A) was compared with the initial amount of the sample to calculate a weight percentage of the "room-temperature xylene-soluble component" in the component (A) (hereinafter referred to as "As(%)"). The amount of the "room-temperature xylene-soluble component" contained in the whole polymers produced was measured by the same method as above to calculate a weight percentage thereof (hereinafter referred to as "CXS(P)(%)").

Then, the weight percentage of the "room-temperature xylene-soluble component" in the component (A) based on the weight of the whole polymers (hereinafter referred to as "CXS(A)(%)") and the weight percentage of the "room-temperature xylene-soluble component" in the component (B) based on the weight of the whole polymers (hereinafter referred to as "CXS(B)(%)") were calculated according to the formulas "As x A/100" and "CXS(P) - CXS(A)", respectively.

Whereas, the weight percentage of the "room-temperature xylene-insoluble component" in the component (A) based on the weight of the whole polymers (hereinafter referred to as "CXIS (A)(%)") was calculated according to the formula "A(%) - CXS (A)(%)". Similarly, the weight percentage of the "room-temperature xylene-insoluble component" in the component (B) based on the weight of the whole polymers (hereinafter referred to as "CXIS (B)(%)") was calculated according to the formula "B(%) - CXS(B)(%)".

<u>(3) Isotactic index of component (A):</u>

The polymer produced after the first-stage polymerization (component (A)) was sampled by the same method as in the above (2), and extracted with n-heptane using a Soxhlet's extractor to measure a weight of the resultant residue and calculate a weight percentage thereof relative to the initial weight of the sample.

<u>(4) Ethylene content in "room-temperature xylene-soluble component" in the component (B):</u>

The "room-temperature xylene-soluble component" contained in the polymer produced after the first-stage polymerization in the above (2) and the "room-temperature xylene-soluble component" contained in the whole polymers were subjected to $^{13}$C-NMR spectrum analysis by the method described in Kang-Bond Lee, et al., "Polymer J.", 28, pp. 696-702 (1996) to measure the ethylene contents in the respective "room-temperature xylene-soluble components" (hereinafter referred to as E(A) and E(P), respectively; unit: % by weight). Based on the measured values, the ethylene content in "room-temperature xylene-soluble component" contained in the component (B) was calculated according to the following formula:

$$[E(P) - E(A) \times (CXS(A)/CXS(P))]/[CXS(B)/CXS(P)]$$

<u><Evaluation of propylene-based resin composition></u>

<u>(1) Preparation of test sample:</u>

0.1 part by weight of tetrakis[methylene-3-(3',5'-di-t-butyl-4'-hydroxyphenyl)propionate]methane as an antioxidant ("IRGANOX 1010" produced by Chiba Specialty Chemicals Corp.) and 0.02 part by weight of hydrotalcite as a neutralizing agent ("ALKAMIZER DHT-4A" produced by Kyowa Kagaku Co., Ltd.) were added to 100 parts by weight of the propylene-based resin composition. The resultant mixture was melt-kneaded using a twin-screw extruder ("PCM45" manufactured by Ikegai Tekko Co., Ltd.) having a cylinder diameter of 45 mm at a set temperature of 200°C and then extruded to form pellets. Then, the obtained pellets were injection molded using an injection-molding machine ("N-100" manufactured by Nippon Seikosho Co., Ltd.) having a mold-clamping pressure of 100 tons at a under-hopper temperature of 175°C, a cylinder temperature of 220°C, a nozzle temperature of 210°C and a mold temperature of 40°C to prepare a test sample.

(2) Melting point peak temperature:

The melting point peak temperature was measured at a temperature rise rate of 10°C/min using a differential scanning colorimeter ("DSC" manufactured by Seiko Instruments Co., Ltd.) according to JIS K7121.

(3) Tensile stress at yielding point:

Measured at a temperature of 23°C and a pulling speed of 50 mm/min using a No. 2-type test piece according to JIS K7113.

(4) Flexural modulus:

Measured at 23°C according to JIS K7203.

<Evaluation of laminated sheet>

(1) Tensile modulus:

The laminated sheet described in detail below was tested using a No. 2-type test piece to measure its tensile modulus at a temperature of 23°C and a pulling speed of 0.5 mm/min according to JIS K7113.

(2) Transparency before and after high-temperature high-pressure sterilization treatment:

The multi-chamber container described in detail below was placed in a high-temperature high-pressure processed sterilization tester ("RCS/40RTGN Model" manufactured by Nichihan Seisakusho Co., Ltd.) and held under pressure. Then, the temperature of an atmosphere in the tester was raised up to 121°C and held at the same temperature for 30 minutes, thereby subjecting the container to sterilization treatment under high-temperature and high-pressure. After completion of the treatment, the multi-chamber container was taken out of the tester and cut into a 250 μm-thick film piece to measure a haze thereof. The thus measured haze of the film piece was compared with that before the sterilization treatment. Meanwhile, the haze was measured according to JIS K6717.

Example 1:

<Production of propylene-based resin composition>

(1) Production of solid component catalyst:

20 liters of dehydrated and deoxygenated n-heptane was introduced into a 50-liter reaction vessel equipped with a stirrer which was previously purged with nitrogen. Then, 4 mol of magnesium chloride and 8 mol of tetrabutoxytitanium were introduced into the reaction vessel, and the contents of the reaction vessel were reacted with each other at 95°C for 2 hours. The obtained reaction mixture was cooled to 40°C, mixed with 480 ml of methylhydropolysiloxane having a viscosity of 20 centistokes, and further reacted for 3 hours. Thereafter, the obtained reaction solution was taken out of the reaction vessel to separate therefrom a solid component produced. The thus obtained solid component was washed with n-heptane.

Successively, 15 liters of dehydrated and deoxygenated n-heptane was introduced into the same type reaction vessel with a stirrer as used above. Then, 3 mol of the above obtained solid component in terms of magnesium atom was added to the reaction vessel, and further a mixed solution prepared by adding 8 mol of silicon tetrachloride to 25 ml of n-heptane was introduced into the reaction vessel at 30°C for 30 minutes. The resultant mixture was heated to 90°C and reacted for one hour. Then, the obtained reaction solution was taken out of the reaction vessel to separate therefrom a solid component produced. The thus obtained solid component was washed with n-heptane.

Further, 5 liters of dehydrated and deoxygenated n-heptane was introduced into the same type reaction vessel with a stirrer as used above. Then, 250 g of the above-prepared silicon tetrachloride-treated titanium-containing solid component, 750 g of 1,5-hexadiene, 130 ml of t-butyl-methyl-dimethoxysilane, 10 ml of divinyldimethylsilane and 225 g of triethyl aluminum were respectively introduced into the reaction vessel, and the contents thereof were contacted with each other at 30°C for 2 hours. Thereafter, the obtained reaction solution was taken out of the reaction vessel to separate therefrom a solid component produced. The thus obtained solid component was washed with n-heptane to obtain a solid component catalyst. As a result, it was confirmed that the obtained solid component catalyst provided a prepolymerized 1,5-hexadiene in an amount of 2.97 g per the titanium-containing solid component added.

(2) Two-stage polymerization of propylene/propylene-ethylene:

A 550-liter first-stage reactor which was maintained at 70°C under pressure (about 3.2 MPa at 70°C) was continuously supplied with propylene, triethyl aluminum and the above solid component catalyst. The solid component catalyst was supplied in such an amount that the polymer production rate was 20 kg/hr. Further, hydrogen as a molecular weight modifier was continuously supplied to the reactor to conduct the first-stage polymerization in a liquid phase.

Successively, the resultant polymer was introduced through a propylene-purged vessel into a 1,900-liter second-stage reactor, and further propylene and ethylene were continuously supplied thereto in such amounts capable of controlling the reaction pressure to 3.0 MPa at 60°C and achieving the composition ratio of the aimed copolymer. Further, hydrogen as a molecular weight modifier was continuously supplied to the reactor, and simultaneously an active hydrogen compound (ethanol) was supplied thereto in an amount of 200 mol per mol of titanium atom contained in the solid component catalyst supplied at the first-stage polymerization and 2.5 mol per mol of the triethyl aluminum, thereby conducting the polymerization in a gas phase. The resultant polymer was continuously transferred to a vessel, and then a moisture-containing nitrogen gas was introduced into the reactor to terminate the reaction (second-stage polymerization).

The propylene-based resin composition obtained after completion of the above second-stage polymerization was kneaded at a temperature of 190 to 210°C using a twin-screw kneading extruder, and then extruded therefrom into strands. The thus extruded strands were water-cooled, cut and then dried to form pellets. The results of the composition analysis and evaluation of the obtained propylene-based resin composition are shown in Table 1 below.

<Preparation of laminated sheet>

A three-layer tubular sheet (inflated sheet) was produced by using the propylene-based resin composition as a material of an inner layer, a composition prepared by kneading the propylene-based resin composition with a styrene-based elastomer ("DYNARON 2320P" produced by JSR Co., Ltd.) at a weight ratio of 70/30 as a material of an intermediate layer, and a composition prepared by kneading the propylene-based resin composition with a propylene-ethylene random copolymer (ethylene content: 2.5% by weight) at a weight ratio of 60/40 as a material of an outer layer.

In the above production process, the respective raw pellets used for formation of the above inner, intermediate and outer layers were supplied to a multilayer-forming circular die (inflation die), extruded into a tubular sheet at a temperature of 180 to 230°C, cooled using a water-cooling ring, and then taken up into a rolled sheet having a thickness of 200 $\mu$m and a lay flat width of 200 mm. Meanwhile, it was confirmed that the thicknesses of the respective layers of the obtained sheet were 40 $\mu$m for the inner layer, 120 $\mu$m for the intermediate layer, and about 40 $\mu$m for the outer layer.

<Bag-making process for production of multi-chamber container>

The above-prepared laminated sheet was formed into a bag shape in order to produce a medical multi-chamber container (L1: 350 mm; L2: 200 mm) as shown in Fig. 1. The bag-making process was conducted as follows.

(i) The sheet was interposed at its predetermined positions between a pair of segments of a heat-sealing mold heated to 160°C under a pressing force of 4 kgf/cm$^2$ for about 4 seconds to form strong sealed portions (3a) to (3c).
(ii) The sheet was interposed at its predetermined position between a pair of segments of a heat-sealing mold heated to 130°C under a pressing force of 4 kgf/cm$^2$ for about 4 seconds to form a weak sealed portion (4).
(iii) The thus heat-sealed sheet was cut at intervals of 350 mm.
(iv) A ship-shaped port member (7) (comprising polypropylene) was inserted into an upper end of the cut sheet, and strongly heat-sealed thereto.

<Filling and heat- and pressure-sterilization of liquid drug>

1,400 ml of a glucose solution and 600 ml of a sodium bicarbonate solution were filled into accommodation chambers (A) and (B) of the above medical multi-chamber container, respectively, and respective filling ports for liquid drug were closed by heat-sealing. Then, the multi-chamber container in which the liquid drug was enclosed, was placed in a heat- and pressure-sterilization apparatus, and the contents thereof were sterilized therein at 121°C for 60 minutes.

<u><Evaluation of multi-chamber container></u>

As a result, it was confirmed that the above medical multi-chamber container was excellent in flexibility and transparency, and had such a heat resistance capable of withstanding the heat- and pressure-sterilization at 121°C. The multi-chamber container was further tested according to the Japan Pharmaceutical Codex general testing method "Testing Method for Plastics for Transfusion" to determine its heavy metal content and eluted substances. As a result, it was confirmed that the medical multi-chamber container had a high safety capable of complying with standards of the Japan Pharmaceutical Codex. The results of measurement for the transparency (haze) of the multi-chamber container before and after the high-temperature high-pressure sterilization treatment are shown in Table 2 below together with the results of measurement for tensile modulus of the laminated sheet.

Next, the seal strength of the multi-chamber container was measured by the following procedures. First, the weak sealed portion (4) and the strong sealed portions (3a) to (3c) of the medical multi-chamber container were respectively cut into a strap shape having a length of 45 mm (including 5 mm sealed portion and 40 mm non-sealed portion) and a width of 15 mm to prepare test pieces. Then, the thus prepared test pieces were respectively tested using a tensile tester (small-size table tester "EZ Test" manufactured by Shimadzu Seisakusho Co., Ltd.) to measure a 180° peel strength thereof at a pulling speed of 200 mm/min. As a result, it was confirmed that the weak sealed portion exhibited a peak value (maximum value) of peel (tensile) strength of 0.6 kgf/15 mm, and the strong sealed portions exhibited a peak value (maximum value) of peel (tensile) strength of 4.1 kgf/15 mm. More specifically, it was recognized that the weak sealed portion had an appropriate peelability, and the strong sealed portions had a sufficient strength, and that the difference in seal strength between the weak sealed portion and the strong sealed portions was as large as 3.5 kgf/ 15 mm which was not achievable by conventional multi-chamber containers.

<u><Example of Heat-Sealing Test></u>

The laminated sheet obtained in Example 1 was heat-sealed by the same method as defined in Example 1 except for using such heat-sealing conditions in which although a pressure of 4 kgf/cm$^2$ and a sealing time of about 5 seconds were kept substantially unchanged, the heat-sealing temperature (surface temperature of heat-sealing mold) was changed from 110 to 220°C at intervals of 10°C, thereby forming 12 kinds of sealed portions. The thus heat-sealed sheet was placed in an autoclave and heat-pressurized at 121°C for 60 minutes. Thereafter, the test pieces were prepared from the sheet and tested to measure a heat-seal strength thereof by the same method as defined in Example 1. The results of the measurement are shown in the column "Example 1" of Table 3 below.

<u>Comparative Example 1:</u>

The same multi-stage polymerization procedure as defined in Example 1 was conducted except that upon preparation of the propylene-based resin composition, the amounts of the raw monomers (such as propylene) charged were mainly varied such that the weight percentages of the "room-temperature xylene-soluble component", the "room-temperature xylene-insoluble component", etc., were different from those of the propylene-based resin composition obtained in Example 1, thereby obtaining a propylene-based resin composition. Then, a laminated sheet was produced by the same method as defined in Example 1 except that the thus obtained propylene-based resin composition was used as a material of the heat-seal layer.

The above-prepared laminated sheet was successively subjected to bag-making process for production of the multi-chamber container, filling with liquid drug and heat- and pressure-sterilization, measurement for transparency (haze) of the multi-chamber container before and after high-temperature high-pressure sterilization treatment, and heat-sealing test, by the same method as defined in Example 1 except that the heat-sealing temperature was changed from 160 to 200°C at intervals of 10°C, thereby forming 5 kinds of sealed portions. The results of measurement for transparency of the sealed portions are shown in Table 2 below together with the results of measurement for tensile modulus of the laminated sheet. Also, the results of the heat-sealing test are shown in the column "Comparative Example 1" of Table 3 below.

<u>Table 1</u>

| <Composition analysis and evaluation of propylene-based resin composition> | | |
|---|---|---|
| Item | Results | |
| | Example 1 | Comp. Example 1 |
| Content of component (A) (%) | 40.6 | 8.3 |

(continued)

| <Composition analysis and evaluation of propylene-based resin composition> | | |
|---|---|---|
| Item | Results | |
| | Example 1 | Comp. Example 1 |
| Content of "room-temperature xylene-insoluble component" in component (A) (%) | 40.4 | 8.1 |
| Content of "room-temperature xylene-soluble component" in component (A) (%) | 0.2 | 0.2 |
| Isotactic index of component (A) (%) | 98.6 | 96.5 |
| Content of component (B) (%) | 59.4 | 91.7 |
| Content of "room-temperature xylene-insoluble component" in component (B) (%) | 24.9 | 79.3 |
| Content of "room-temperature xylene-soluble component" in component (B) (%) | 34.5 | 12.4 |
| Ethylene content in "room-temperature xylene-insoluble component" in component (B) (%) | 18.7 | 5.5 |
| Propylene content in polymer (%) | 89.8 | 94.5 |
| "$\alpha t$" (%) | 10.2 | 5.5 |
| "CXS" (%) | 34.7 | 12.6 |
| $5\alpha t-25$ | 26.0 | 2.5 |
| Melting point peak temperature (°C) | 164.2 | 134.6 |
| Tensile stress at yielding point (MPa) | 12.8 | 16.8 |
| Flexural modulus (MPa) | 318 | 482 |

Table 2

| <Evaluation of laminated sheet> | | |
|---|---|---|
| Item | Results | |
| | Example 1 | Comparative Example 1 |
| Tensile modulus (MPa) | 230 | 350 |
| Transparency (haze) before high-temperature high-pressure sterilization treatment (%) | 3 | 6 |
| Transparency (haze) after high-temperature high-pressure sterilization treatment (%) | 3 | 8 |

Table 3

| <Results of heat-sealing test> | | |
|---|---|---|
| Heat-sealing temperature (°C) | Seal strength (Kgf/15 mm) | |
| | Example 1 | Comp. Example 1 |
| 110 | 0.1 (lack of seal strength) | - |
| 120 | 0.2 (weak seal) | - |
| 130 | 0.5 (weak seal) | - |

(continued)

| <Results of heat-sealing test> | | |
|---|---|---|
| Heat-sealing temperature (°C) | Seal strength (Kgf/15 mm) | |
| | Example 1 | Comp. Example 1 |
| 140 | 1.2 (weak seal) | - |
| 150 | 3.5 (strong seal) | - |
| 160 | 4.3 (strong seal) | 0.1 (lack of seal strength) |
| 170 | 4.8 (strong seal) | 0.2 (weak seal) |
| 180 | 5.0 (strong seal) | 3.9 (strong seal) |
| 190 | 4.2 (strong seal) | 3.8 (strong seal) |
| 200 | 4.0 (strong seal) | 3.6 (strong seal) |
| 210 | 4.0 (strong seal) | - |
| 220 | 4.2 (strong seal) | - |
| T1 | 120 | 170 |
| T2 | 145 | 175 |
| T2-T1 | 25 | 5 |
| Note: T1: Heat-sealing temperature capable of providing a heat-seal strength of 0.2 kgf/15 mm. T2: Heat-sealing temperature capable of providing a heat-seal strength of 2.0 kgf/15 mm. | | |

The following facts were apparently confirmed from the measurement results shown in Table 3. That is, in the case of the propylene-based resin composition used in the present invention, the heat-sealing temperature and the seal strength were substantially proportional to each other in a broad heat-sealing temperature range of 120 to 180°C (temperature width: 60°C). Therefore, the heat-sealing temperature range capable of forming the weak sealed portion was also as broad as 120 to 140°C (temperature width: 20°C), so that the seal strength could be readily controlled by changing the heat-sealing temperature. In addition, since both the temperature ranges capable of forming the weak sealed and strong sealed portions were broad, even though the heat-sealing temperature was fluctuated to some extent, it was possible to form the respective heat-sealed portions having the desired seal strength. On the contrary, in the case of the conventional resin compositions, the heat-sealing temperature capable of forming the weak sealed portion was limited to a narrower range near 170°C. Therefore, the slight change in heat-sealing temperature caused extreme change in seal strength, so that it was difficult to control a seal strength of the weak sealed portion.

INDUSTRIAL APPLICABILITY

As described above, according to the present invention, there is provided a multi-chamber container comprising a resin film or sheet formed into a bag shape by heat-sealing and having a partition portion provided therein wherein the difference in heat-seal strength between the partition portion and peripheral portions of the container can be increased as compared to conventional containers. Further, upon formation of the partition portion, it is possible to easily control the temperature of a heat-sealing mold. In addition, the multi-chamber container of the present invention is excellent in safety, flexibility, transparency and heat resistance and, therefore, can be suitably used in medical applications. Thus, the present invention is remarkably valuable from the industrial viewpoint.

**Claims**

1.  A multi-chamber container comprising a resin film or sheet formed into a bag shape by heat-sealing and including a partition portion formed therein, said multi-chamber container including a heat-sealed portion comprising a propylene-based resin composition wherein both of the following requirements [I] and [II] are satisfied:

    [I] the propylene-based resin composition contains the following component (A) obtained by a first-stage polymerization and the following component (B) obtained by a second-stage polymerization such that the component (A) is contained in an amount of 10 to 60% by weight and the component (B) is contained in an amount of 40 to 90% by weight, based on the total weight of the components (A) and (B):

Component (A): a polymer component containing propylene having an isotactic index, as measured according to the present specification, of not less than 90 % as a main component; and Component (B): a copolymer component comprising a copolymer produced from propylene and an $\alpha$-olefin other than propylene having not more than 8 carbon atoms with the proviso that propylene and ethylene are contained therein as essential components, and containing a room-temperature xylene-insoluble component in an amount of from more than 20% by weight to 70% by weight based on the total weight of the whole polymers as the sum of the components (A) and (B), and a room-temperature xylene-soluble component in an amount of from 10 to 60% by weight based on the total weight of the whole polymers as the sum of the components (A) and (B), said room-temperature xylene-soluble component having a content of the $\alpha$-olefin other than propylene of less than 20% by weight;

[II] the propylene-based resin composition satisfies the following requirements (i) to (iv):

(i) the propylene content thereof is within the range of 85 to 95% by weight;
(ii) the content of an $\alpha$-olefin other than propylene (hereinafter referred to merely as "$\alpha t$"; unit: % by weight) and the content of a room-temperature xylene-soluble component (hereinafter referred to merely as "CXS") therein satisfy the following formula:

$$CXS > 5\alpha t - 25$$

wherein $\alpha t$ is 5 to 15% by weight;
(iii) the melting point peak temperature thereof is not less than 160°Cas measured at a temperature rise rate of 10 °C/min using DSC according to JIS K7121; and
(iv) the tensile stress at the yielding point thereof is not more than 15 MPa, as measured according to ISO-R1184.

2. The multi-chamber container according to claim 1,
wherein the resin films or sheets constituting the partition portion are peelable off from each other.

3. The multi-chamber container according to claim 1 or 2, wherein the resin film or sheet includes three or more layers comprising an innermost layer comprising the propylene-based resin composition, an intermediate layer comprising a resin composition containing the propylene-based resin composition and a styrene-based elastomer, and an outermost layer comprising a propylene random copolymer resin or a resin composition containing the propylene-based resin composition and a propylene random copolymer resin.

4. The multi-chamber container according to any one of claims 1 to 3, wherein the propylene-based resin composition satisfies such a requirement that the difference between the heat-sealing temperature (T1) (°C) providing a heat-seal strength of 0.2 kgf/15 mm and the heat-sealing temperature (T2) (°C) providing a heat-seal strength of 2.0 kgf/15 mm (T2 - T1) is not less than 20°C, and exhibiting a heat-seal strength of not less than 3 kgf/15 mm, as measured according to the present specification.

**Patentansprüche**

1. Mehrkammerbehälter, der einen Harzfilm odereine Harzfolie, der/die durch Wärmeversiegelung in einer Taschenform ausgebildet ist, enthält und einen darin gebildeten Trennabschnitt beinhaltet, wobei der Mehrkammerbehälter einen wärmeversiegelten Teil beinhaltet, der eine Propylen-basierte Harzzusammensetzung enthält, wobei die folgenden beiden Voraussetzungen [I] und [II] erfüllt sind:

[I] die Propylen-basierte Harzzusammensetzung enthält den folgenden Bestandteil (A), der durch eine Erststufen-Polymerisation erhalten wird, und den folgendem Bestandteil (B), der durch eine Zweitstufen-Polymerisation erhalten wird, so dass der Bestandteil (A) in einer Menge von 10 bis 60 Gew.% enthalten ist und der Bestandteil (B) in einer Menge von 40 bis 90 Gew.% enthalten ist, bezogen auf das Gesamtgewicht der Bestandteile (A) und (B):

Bestandteil (A): Polymerbestandteil, der Propylen mit einem isotaktischen Index, gemessen gemäß der vorliegenden Patentschritt, von nicht weniger als 90 % als Hauptbestandteil enthält; und

Bestandteil (B): Copolymerbestandteil, der ein Copolymer, das aus Propylen und einem von Propylen verschiedenen $\alpha$-Olefin mit nicht mehr als 8 Kohlenstoffatomen hergestellt ist, enthält, mit der Maßgabe, dass Propylen und Ethylen darin als wesentliche Bestandteile enthalten sind, und der einen bei Raumtemperatur in Xylol unlöslichen Bestandteil in einer Menge von mehr als 20 Gew.% bis 70 Gew.%, bezogen auf das Gesamtgewicht der gesamten Polymere als Summe der Bestandteile (A) und (B), und einen bei Raumtemperatur in Xylol löslichen Bestandteil in einer Menge von 10 bis 60 Gew.%, bezogen auf das Gesamtgewicht der gesamten Polymere als Summe der Bestandteile (A) und (B) enthält, wobei der bei Raumtemperatur in Xylol lösliche Bestandteil einen Gehalt des von Propylen verschiedenen $\alpha$-Olefins von weniger als 20 Gew.% aufweist;

[II] die Propylen-basierte Harzzusammensetzung erfüllt die folgenden Voraussetzungen (i) bis (iv):

(i) der Propylengehalt darin liegt im Bereich von 85 bis 95 Gew.%;
(ii) der Gehalt des von Propylen verschiedenen $\alpha$-Olefins (nachstehend lediglich als "$\alpha$t"; Einheit: Gew.% bezeichnet) und der Gehalt des bei Raumtemperatur in Xylol löslichen Bestandteils (nachstehend lediglich als "CXS" bezeichnet) darin erfüllt die folgende Formel:

$$CXS > 5\alpha t - 25$$

wobei $\alpha$t 5 bis 15 Gew.% ist;
(iii) die Schmelzpunkt-Peaktemperatur darin beträgt nicht weniger als 160°C, gemessen bei einer Temperaturerhöhungsrate von 10°C/min unter Verwendung von DSC gemäß JIS K7121; und
(iv) die Zugspannung an der Streckgrenze darin beträgt nicht mehr als 15 MPa, gemessen gemäß ISO-R1184.

2. Mehrkammerbehälter gemäß Anspruch 1, wobei die Harzfilme oder Harzfolien, die den Trennabschnitt bilden, voneinander abziehbar sind.

3. Mehrkammerbehälter gemäß Anspruch 1 oder 2, wobei der Harzfilm oder die Harzfolie drei oder mehr Schichten umfasst, die eine innerste Schicht mit der Propylenbasierten Harzzusammensetzung, eine intermediäre Schicht mit einer Harzzusammensetzung, die die Propylen-basierte Harzzusammensetzung und ein Styrol-basiertes Elastomer enthält, und eine äußerste Schicht mit einem statistischen Propylen-Copolymerharz oder einer Harzzusammensetzung, die die Propylen-basierte Harzzusammensetzung und ein statistisches Propylen-Copolymerharz enthält, umfassen.

4. Mehrkammerbehälter gemäß einem der Ansprüche 1 bis 3, wobei die Propylen-basierte Harzzusammensetzung eine solche Voraussetzung erfüllt, dass die Differenz zwischen der Wärmeversiegelungstemperatur (T1) (°C), die eine Wärmeversiegelungsstärke von 0,2 kgf/15 mm liefert, und der Wärmeversiegelungstemperatur (T2) (°C), die eine Wärmeversiegelungsstärke von 2,0 kgf/15 mm liefert, (T2 - TI) nicht weniger als 20°C beträgt, und wobei die Propylen-basierte Harzzusammensetzung eine Wärmeversiegelungsstärke von nicht weniger als 3 kgf/15 mm, gemessen gemäß der vorliegenden Patentschrift, aufweist.

**Revendications**

1. Conteneur à multiples chambres comprenant un film ou une feuille de résine formée en une forme de sac par cachetage à chaud et incluant une partie de séparation formée en son sein, ledit conteneur à multiples chambres incluant une partie cachetée à chaud comprenant une composition de résine à base de propylène dans laquelle les deux exigences suivantes [I] et [II] sont satisfaites :

[I] la composition de résine à base de propylène contient le composant suivant (A) obtenu par une polymérisation de première étape et le composant suivant (B) obtenu par une polymérisation de seconde étape de sorte que le composant (A) est contenu en une quantité de 10 à 60 % en poids et le composant (B) est contenu en une quantité de 40 à 90 % en poids, en se basant sur le poids total des composants (A) et (B) :

Composant (A) : un composant de polymère contenant du propylène ayant un indice isotactique, tel que mesuré selon la présente spécification, non inférieur à 90 % en tant que composant principal ; et

Composant (B) : un composant de copolymère comprenant un copolymère produit à partir de propylène et d'une $\alpha$-oléfine autre que du propylène n'ayant pas plus de 8 atomes de carbone avec la clause restrictive que du propylène et de l'éthylène sont contenus en son sein en tant que composants essentiels, et contenant un composant insoluble dans le xylène à température ambiante en une quantité allant de plus de 20 % en poids jusqu'à 70 % en poids en se basant sur le poids total des polymères entiers en tant que somme des composants (A) et (B), et un composant soluble dans le xylène à température ambiante en une quantité de 10 à 60 % en poids en se basant sur le poids total des polymères entiers en tant que somme des composants (A) et (B), ledit composant soluble dans le xylène à température ambiante ayant une teneur de l'$\alpha$-oléfine autre que du propylène inférieure à 20 % en poids ;

[II] la composition de résine à base de propylène satisfait aux exigences suivantes (i) à (iv) :

(i) la teneur en propylène de celle-ci est dans la plage de 85 à 95 % en poids ;
(ii) la teneur d'une $\alpha$-oléfine autre que du propylène (mentionné simplement dans la suite du document par « $\alpha$t » ; unité : % en poids) et la teneur d'un composant soluble dans le xylène à température ambiante (mentionné simplement dans la suite du document par « CXS ») en son sein, satisfont à la formule suivante :

$$CXS > 5\alpha t - 25$$

dans laquelle $\alpha$t est de 5 à 15 % en poids ;
(iii) la température maximale de point de fusion de celle-ci n'est pas inférieure à 160°C, telle que mesurée à un taux d'élévation de température de 10°C/min en utilisant une DSC selon JIS K7121 ; et
(iv) la contrainte de traction à la limite élastique de celle-ci n'est pas supérieure à 15 MPa, telle que mesurée selon ISO-R1184.

2. Conteneur à multiples chambres selon la revendication 1, dans lequel les films ou les feuilles de résine constituant la partie de séparation peuvent être décollées les unes des autres.

3. Conteneur à multiples chambres selon la revendication 1 ou 2, dans lequel le film ou la feuille de résine inclut trois couches ou plus comprenant une couche la plus intérieure comprenant la composition de résine à base de propylène, une couche intermédiaire comprenant une composition de résine contenant la composition de résine à base de propylène et un élastomère à base de styrène, et une couche la plus extérieure comprenant une résine de copolymère statistique de propylène ou une composition de résine contenant la composition de résine à base de propylène et une résine de copolymère statistique de propylène.

4. Conteneur à multiples chambres selon l'une quelconque des revendications 1 à 3, dans lequel la composition de résine à base de propylène satisfait à une exigence telle que la différence entre la température de cachetage à chaud (T1) (°C) donnant une force de cachetage à chaud de 0,2 kgf/15 mm et la température de cachetage à chaud (T2) (°C) donnant une force de cachetage à chaud de 2,0 kgf/15 mm (T2 - T1) n'est pas inférieure à 20°C, et présentant une force de cachetage à chaud non inférieure à 3 kgf/15 mm, telle que mesurée selon la présente spécification.

## Fig. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 56053062 A
- JP 57105272 A
- JP 3066845 A
- JP 6026563 A
- JP 63309263 A
- JP 1240469 A
- JP 2004671 A
- JP 2057584 A
- JP 2241457 A
- JP 2255418 A
- JP 4242647 A
- JP 5031153 A
- JP 5068702 A
- JP 8131515 A

- JP 8229100 A
- JP 2000070331 A
- JP 2000005276 A
- US 5176634 A
- WO 0135898 A
- EP 0483523 A1
- US 4211852 A
- JP 4023798 B
- JP 2764746 B
- JP 428704 A
- JP 436636 A
- JP 59133203 A
- JP 60079005 A

**Non-patent literature cited in the description**

- **KANG-BOND LEE et al.** *Polymer J.,* 1996, vol. 28, 696-702